# EUROPEAN PATENT APPLICATION

(11) **EP 1 716 751 A1**
(43) Date of publication of application: **02.11.2006**
(21) Application number: 05009342.6
(22) Date of filing: 28.04.2005
(51) Int. Cl.: A01K 67/00

(54) **Animal models for cell therapy and cell based gene therapy**

(71) Applicant: Veterinärmedizinische Universität Wien, 1210 Vienna (AT)
(72) Inventor: Erben, Reinhold G., 1210 Vienna (AT)
(74) Representative: Sonn & Partner Patentanwälte

(57) **Abstract**

The present invention describes the use of marker tolerant animals for the study of cell based therapies.

## Description

The present invention relates to diagnostic models for cell therapy and cell based gene therapy.

Cell and gene therapy are thought to revolutionise tissue repair in various organs, cancer therapy, and therapy of genetic diseases in the near future (1). Therefore, cell and gene therapy holds great promise to cure or ameliorate a large variety of diseases of various organ systems. Cell therapy involves the syngeneic (genetically identical), allogeneic (different individuals of the same species), or xenogeneic (between species) transplantation of cells for therapeutic purposes.

Potential therapeutic targets for cell and gene therapy are:
- Liver diseases (for example cell therapy of liver cirrhosis or other severe liver diseases)
- Lung diseases (for example cell-based gene therapy of cystic fibrosis)
- Skin diseases (for example cell therapy of skin defects with epidermal cells)
- Cardiovascular diseases (for example cell therapy of myocardial infarction with mesenchymal cells, gene therapy of cardiomyopathies, for example with vascular growth factors)
- Kidney diseases (for example cell therapy or cell-based gene therapy of glomerulonephritis or of renal failure)
- Orthopedic diseases (for example cell therapy of osteoarth ritis, disc degeneration, ligament injuries, or cell-based gene therapy of genetic myopathies)
- Diabetes mellitus (for example cell therapy of diabetes by transplantation of autologous beta cells grown from precurs or cells extracted from pancreas, liver, or bone marrow)
- Diseases of the central nervous system (for example cell therapy or cell-based gene therapy of neurodegenerative diseases such as Parkinson's disease or amyotrophic lateral sclerosis, cell therapy of cerebral infarction (palsy), cell therapy of multiple sclerosis)
- Cancer (for example cell therapy with ex vivo conditioned autologous immune cells, cell-based gene therapy with autologous cells expressing for example immunostimulatory cytokines such as interleukin 2)

It is clear that any thorough evaluation of the efficacy and applicability of cell and gene therapy methods requires animal models that allow tracing the fate of individual transplanted donor or manipulated cells in the host organism. In order to trace individual cells, it is necessary that these cells are somehow labelled. The labelling is usually done by introducing marker genes into the genome of the cells under investigation. Marker genes are genes whose permanent or transient expression can be used to label cells. Marker genes can either be permanently integrated into the genome of transgenic animals so that all or at least some somatic cells are permanently labelled, or normal cells can be transduced with vectors containing the marker gene. Suitable marker genes are for example Escherichia coli lacZ, green fluorescent protein (GFP) from the jellyfish Aequorea victoria, human placental alkaline phosphatase (hPLAP), or the neomycin phosphotransferase.

During recent years, it has become increasingly clear that membrane or even intracellular expression of any foreign protein, and, thus, of any marker gene, will elicit immune-mediated rejection of the cells carrying the marker gene in the recipients (7-11).

Therefore, immune-mediated rejection of genetically altered cells is a general and very significant problem in transplantation studies using cells labelled with any marker gene, and also in gene therapy protocols. This problem has severely hampered the utility of animal models aimed at testing the usefulness of cell and gene therapy especially in long-term studies. There are several solutions to this problem of immune-mediated rejection of transplanted, labelled cells:
The host can be treated with immunosuppressive drugs such as cyclosporins or glucocorticoids (12). It is clear that with this approach it is difficult to tell whether immune-mediated phenomena were actually absent or not. In addition, immunosuppressive drugs have to be introduced into the experimental system, possibly influencing the outcome of the experiments.

Cells of another species (e.g. human cells) can be transplanted into mice or other animals suffering from severe immune defects such as severe combined immunodeficiency (SCID). SCID mice are unable to reject foreign tissues. Problems with this experimental system are that SCID mice are difficult to keep and that they are a highly artificial system due to the severe immune defects.

Accordingly, there is still an urgent need for a model system for observing and studying the performance and effectiveness of a cell based therapy based on genetic molecular markers without being biased by unnatural effects, such as immunologic side effects.

Therefore, the present invention provides the use of marker tolerant animals as a diagnostic model for cell therapy and cell-based therapy.

Since the present invention provides for an animal model, it is clear that the term "animal" exclusively refers to non-human animals as far as the model animal is concerned.

The present solution to the aforementioned problem consists of an entirely different approach as the prior art strategies: In the model which is established by the present invention animals are first made immunologically tolerant to the marker gene that allows tracing of cells to be transplanted into the animals wherein the performance of the cells will be analysed. In a second step, cell and gene therapies with cells labelled with the same marker gene can be tested in the complete absence of immune-mediated rejection. Thus, this model is almost fully equivalent to the situation in human patients using autologous adult stem cells for cell and gene therapy.

In humans, clearly the most practical and applicable method for cell and gene therapy is therapy with autologous stem cells. The general steps of such a therapy can be outlined as follows: Stem cells are extracted from the patient (for example from bone marrow), cultivated and expanded, possibly differentiated in a certain direction or manipulated to express certain genes for gene therapy, and these cells are then re-injected into the same patient either systemically or locally for tissue repair or gene therapy. In addition, tissues or organs can be grown from these cells in culture, and the tissues or organs can be re-transplanted into the patient. Therefore, autologous stem cell therapy is not complicated by immune-mediated rejection phenomena as long as the cells are not transduced with genes encoding non-human proteins. Thus, it can be envisaged that any results obtained with the model according to the present invention are highly predictive of the therapeutic potential of autologous cell and gene therapy in humans.

Recently, it has been shown that low amounts of foreign proteins (for example proteins from fetal bovine serum) attached to the cell surface or internalised during culture can elicit an immune response in the host (13). The present invention (especially the protocols E - G described in the example section) is also capable of coping with this problem. When cells for induction of tolerance and cells for later cell therapy are cultivated in an identical fashion, tolerance will also be induced to any antigens stemming from the culture medium.

The present invention is specifically useful for the evaluation of syngeneic and allogeneic cell therapies. The present invention is most appropriate to study syngeneic and allogeneic cell therapies with mesenchymal stem cells. Cell-based gene therapy involves the transplantation of cells transduced to express certain genes for therapeutic purposes.

Further aspects of the present invention are
- a kit for investigating cell therapy or cell-based gene therapy comprising a marker tolerant animal and a cell comprising the marker and
- a method for studying the applicability of cells in cell-therapy and cell-based gene therapy comprising the steps of providing said cells to be studied with a marker, transplanting said cells with the marker to an animal, said animal comprising a tolerance against said marker, and studying the applicability of said cells with said marker in cell therapy and cell-based gene therapy of said marker tolerant animals, preferably (i.e. usually) comprising killing said animals.

The following preferred embodiments relate to the use, the kit as well as the method according to the present invention, wherever appropriate.

Preferably, the marker tolerant animals according to the present invention are lethally irradiated inbred or cloned animals transplanted with bone marrow cells expressing said marker, animals having been exposed neonatally to said marker or animals having been exposed postnatally to marker cells by intravenous infusion.

Specifically preferred are animals which are provided by the following method steps:
- providing said cells to be studied with a marker,
- transplanting said cells with the marker to an animal, said animal comprising a tolerance against said marker, and
- studying the applicability of said cells with said marker in cell therapy and cell-based gene therapy of said marker tolerant animals, preferably comprising killing said animals.

The marker to be used is preferably a marker which is already established in cell therapy and transgenic animals. It should be easily detectable upon expression and not be harmful in the animal to be studied. Most suitable markers can be selected from Escherichia coli lacZ, green fluorescent protein (GFP), human placental alkaline phosphatase (hPLAP) or neomycin phosphotransferase.

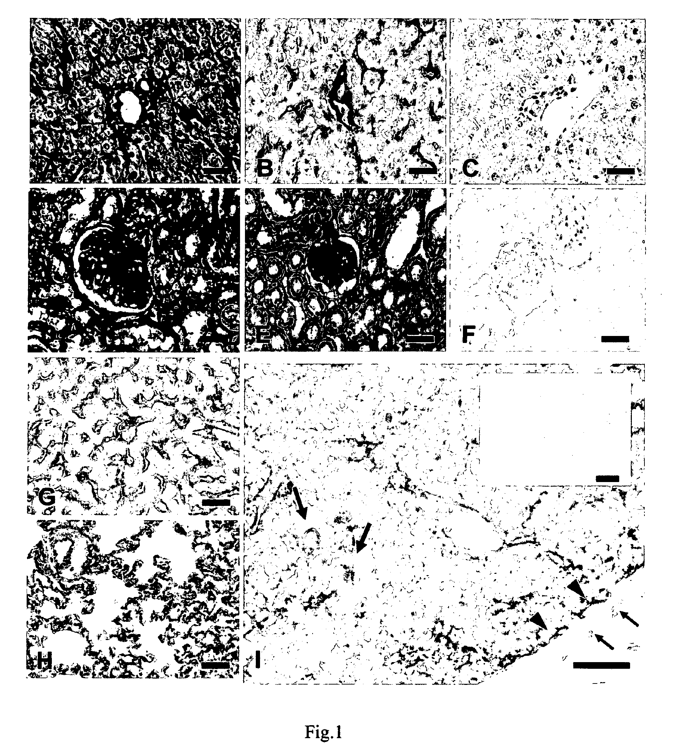

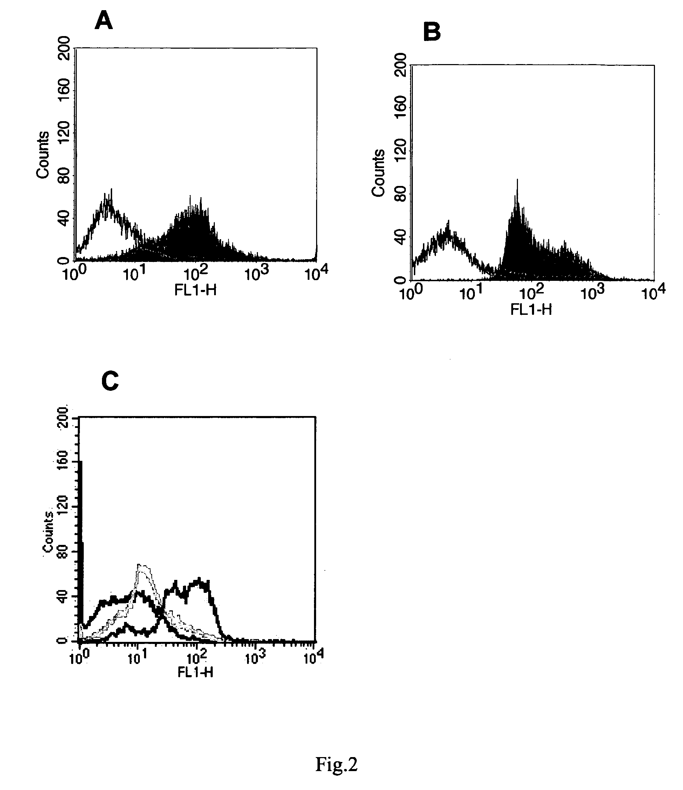

The preferred marker genes for studies involving cell and gene therapy - lacZ, GFP and hPLAP - are known for their suitable detectability in histological sections. LacZ can easily be detected histochemically, but is heat labile, and the enzyme activity is destroyed during paraffin embedding (2). Therefore, pre-embedding staining methods or immunohistochemistry are necessary to visualise the marker lacZ. GFP can easily be visualised in living cells because of its fluorescent properties (3). However, the fluorescent properties of GFP are greatly diminished during normal paraffin embedding, and sensitive detection of GFP in paraffin sections requires either sophisticated equipment (4) or immunohistochemical detection methods (5). Thus, the usefulness of GFP for histological studies is limited. hPLAP is a marker gene highly suitable for histological studies requiring tissue sectioning and excellent tissue morphology, because hPLAP is a heat-stable enzyme that retains its enzymatic activity during routine paraffin embedding (6). In addition, it could be shown with the present invention that hPLAP enzyme activity is preserved during methylmethacrylate (MMA) embedding (Fig. 1). Enzymatic activity of hPLAP can easily and very sensitively be detected histochemically or immunohistochemically in tissue sections by post-embedding procedures (Fig. 1). Therefore, hPLAP is one of the most suitable marker genes to date for the evaluation of cell and gene therapies. On the other hand, it was also found in the course of the present invention that the genetic marker hPLAP induced strong humoral immune responses in wild-type recipient rats, resulting in the rejection of intravenously infused cells from transgenic donors (Fig. 2), also proving that the need for an absolutely marker tolerant animal is important for providing a non-biased model.

The preferred fields of use of the present animal model are cell therapies or cell-based gene therapies for stroke, multiple sclerosis, neurodegenerative disorders, arthroses, intervertebral disc degeneration, ligament injuries, myopathies, myocardial infarction, cardiomyopathies, glomerulonephritis, severe liver cell damages, epidermal cell therapy or cancer, including the generation and transplantation of tissues or organs grown from individual cells in culture.

Preferably, the animal to be used according to the present invention is a rodent, especially a rabbit, a hamster, a mouse, a guinea pig or a rat, a primate, especially a chimpanzee, or a pig.

Depending on the model or on the cells to be studied, the marker in the cell is controlled by a specific promoter. A preferred promoter is an inducible promoter (reviewed by Romano G Systems for regulated or tissue-specific gene expression. Drug News Perspect 17:85-90; Clackson T Regulated gene expression systems. Gene Therapy 2000, 7:120-125; Pollock R and Clackson T Dimerizer-induced gene expression. Current Opin Biotechnol 2002; 13:459-467). In other preferred embodiments, the promoter is a ubiquitous constitutive promoter, especially ROSA26 (R26) promoter, human, rat, or chicken β-actin promoter or β-actin promoter with cytomegalovirus enhancer, cytomegalovirus promoter, ubiquitin promoter, or SV40 promoter. In other preferred embodiments, the promoter is a cell- or tissue-specific constitutive promoter targeting marker expression to specific hematopoietic cells such as the megakaryocytic/platelet-specific alphaIIb promoter (16), the lymphoid cell-specific hCD2 promoter (17) or the macrophage-specific c-fms gene promoter (18). A specific advantage of a constitutive promoter is that all cells derived from a genetically labeled cell express the marker regardless of differentiation status. A specific advantage of an inducible promoter and a cell- or tissue-specific promoter in the current invention is that marker gene expression can be limited in space and/or time. Using cells expressing the marker under control of a tissue-specific or inducible promoter for induction of tolerance, a marker tolerant animal can be generated without background expression of labeled cells in the marker tolerant animal, or with background expression only in cells not interfering with the analysis of applicability. A further specific advantage of an inducible promoter in transplanted cells is that cell-based gene therapy can be monitored if the marker and the therapeutic gene are under the control of the same inducible promoter.

The preferred cells to be applied in the model for cell therapy or cell-based gene therapy according to the present invention is a therapy employing mesenchymal cells, especially osteoblasts, chondrocytes, adipocytes, fibroblasts, myoblasts or combinations thereof.

Other preferred forms of cell therapy or cell-based gene therapy to be studied with the present model are a therapy employing non-hematopoietic cells selected from epithelial cells, endothelial cells, liver cells, pancreas cells, neuronal cells or combinations thereof.

According to a preferred embodiment of the present invention, the cells with the marker are transplanted into deficient parts of the mammal selected from the group consisting of heart, central nervous system (CNS), bone, cartilage, joint, lung, intestine, liver or pancreas.

Preferably, the marker tolerant animal in the model or kit of the present invention is a rodent, especially a rabbit, a hamster, a mouse, a guinea pig or a rat, a primate, especially a chimpanzee, or a pig.

Preferred cells comprising the marker are differentiated or partially differentiated mesenchymal cells, especially mesenchymal cells contained in bone marrow or mesenchymal cells derived from bone marrow stem cells.

According to a preferred embodiment, the kit according to the present invention further comprises 1) a marker tolerant animal, 2) cells comprising the marker, 3) a transfection vector to transduce cells with the marker, 4) a detection system for the marker, for example in histological sections.

In the method for studying the applicability of cells in cell-therapy and cell-based gene therapy according to the present invention, the following steps are applied:
- providing said cells to be studied with a marker,
- transplanting said cells with the marker to an animal, said animal comprising a tolerance against said marker, and
- studying the applicability of said cells with said marker in cell therapy and call-based gene therapy of said marker tolerant animals, preferably comprising killing said animals.

As mentioned above, the marker tolerant animals may be provided by any suitable technique, the most preferred being selected from the group of inbred animals, cloned animals, bone marrow marker cells transplanted animals, animals having been exposed neonatally to said marker or animals having been exposed postnatally to marker cells by intravenous infusion.

The present invention also provides a specific technique for providing excellently suitable marker tolerant animals, wherein the marker tolerant animals are provided by a method which comprises the steps of
- obtaining a transgenic mammal expressing a marker protein under the control of a promoter,
- isolating bone marrow cells (BMCs) from the transgenic mammal,
- irradiating a wild type mammal of the same species and
- injecting the isolated BMCs into the irradiated wild type mammal,
before transplanting said cells with the marker to the wild type mammal.

The animals obtainable by this specific technique also represent an aspect of the present invention. The specific advantage of lethal irradiation followed by bone marrow transplantation in inbred or cloned animals is that this method represents the most robust approach for induction of lifelong and complete immunological tolerance to the marker gene.

The present invention is further described by the following examples and the drawing figures, yet without being restricted thereto.

Fig. 1 shows paraffin and methylmethacrylate (MMA) sections of liver and kidney from R26-hPLAP-transgenic and wild-type rats stained for hPLAP activity after heat pretreatment. In paraffin sections of liver from transgenic rats, hPLAP staining is more intense in specimens fixed with 40% ethanol (A) compared with PFA fixation (B). No staining is observed in ethanol-fixed liver of a wild-type rat (C). The kidney from a transgenic rat shows comparable hPLAP staining intensity in paraffin- (D) and MMA-embedded specimens (E). However, morphological detail is improved in MMA sections, and renal tubular cells show a clear membranous hPLAP staining pattern. Note the ubiquitous hPLAP staining pattern in transgenic tissues. No staining is seen in ethanol-fixed, MMA-embedded kidney of a wild-type rat (F). Sections A - F were stained for hPLAP enzyme activity overnight at room temperature and were counterstained with nuclear fast red. Immunohistochemical detection of hPLAP (G - I) using the monoclonal antibody 8B6 revealed strong hPLAP expression in PFA-fixed and paraffin-embedded liver (G) and lung (H), as well as in the ethanol-fixed, MMA-embedded tibia from a R26-hPLAP-tg rat (I). Bone sections from wild-type rats did not show any staining with 8B6 (inset in I). The intense cytoplasmic staining pattern in megakaryocytes (large arrows), and the strong membranous staining of osteoblasts (arrowheads) and osteocytes (small arrows) in transgenic bone tissue (I) are specifically to be noted. Five-µm-thick sections. Bar = 50 µm.

Fig. 2 shows a flow cytometric analysis of peripheral blood and bone marrow cells from R26-hPLAP-transgenic (tg) rats. The single parameter fluorescence histograms show hPLAP expression (mAb clone 8B6) on the majority of peripheral blood leukocytes (A) and practically all bone marrow cells (B) in R26-hPLAP-tg rats (grey area), but no expression in wild-type rats (white area). When peripheral blood leukocytes from R26-hPLAP-tg rats are incubated with serum from a naive wild-type rat, and stained for bound antibodies, no staining is seen (white area, C). However, serum from the same wild-type intraperitoneally injected with cells from transgenic donors 3 times shows strong reactivity with peripheral blood leukocytes from R26-hPLAP-tg rats (green, after 2 injections; red, after 3 injections), indicating the induction of circulating antibodies against transgenic cells in the recipient. Ten-thousand cells were analysed in each sample.

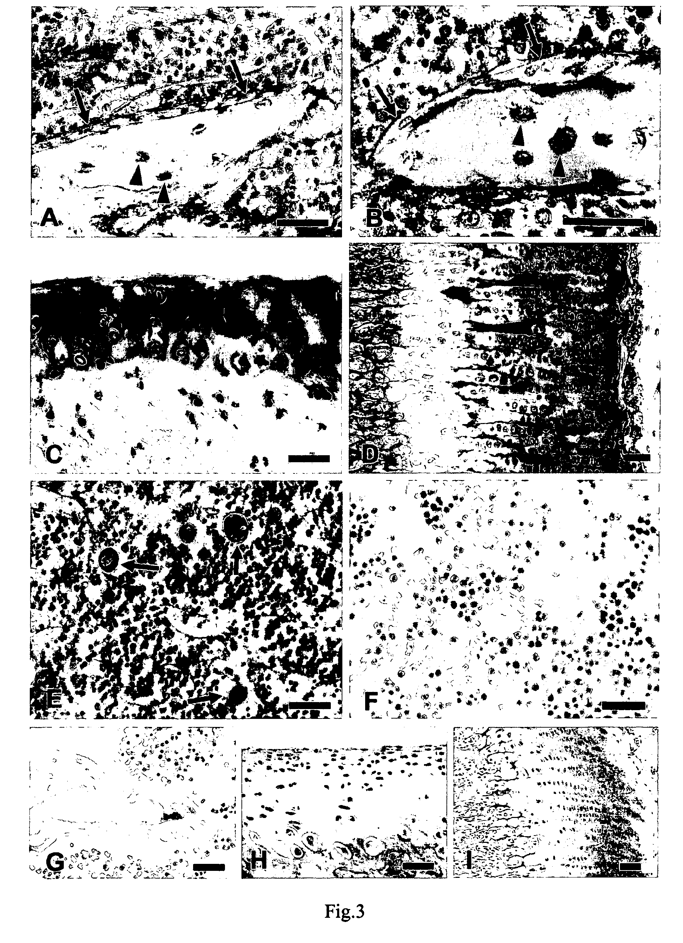

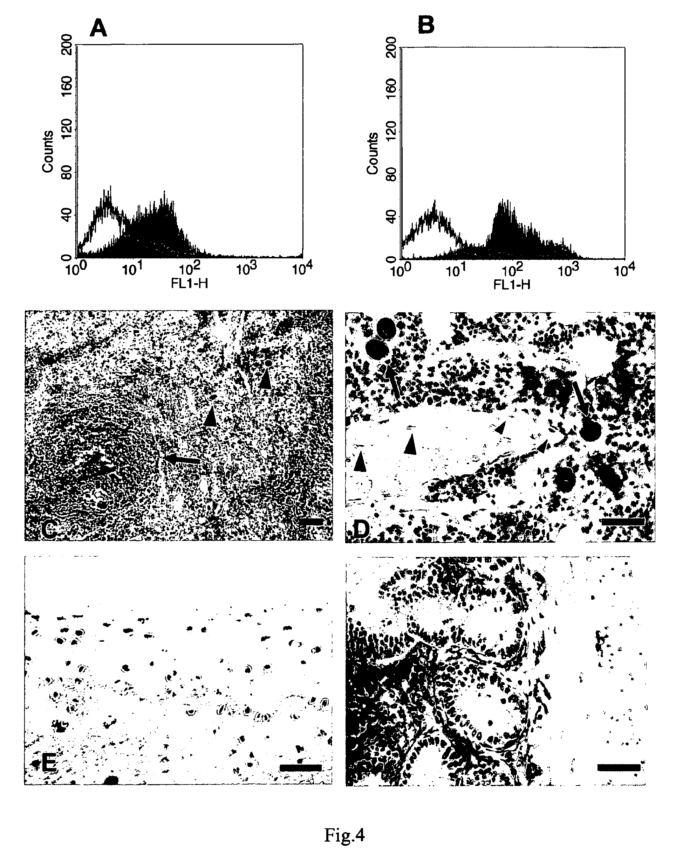

Fig. 3 shows ubiquitous expression of hPLAP in bone, cartilage, and bone marrow cells of R26-hPLAP-transgenic rats (A-E). Osteoblasts (arrows) and osteocytes (arrowheads, A-B) show intense membranous hPLAP staining in transgenic rats (A). Osteoclasts (arrows) display weak membrane expression of hPLAP and absence of cytoplasmic staining (B), whereas chondrocytes in articular and growth plate cartilage are intensely stained (C-D). Hematopoietic bone marrow cells show ubiquitous hPLAP staining with very strong staining on the cell membrane and in the cytoplasm of megakaryocytes (E). No staining is seen in bone marrow (F), bone (G), articular cartilage (H), or growth plate cartilage from wild-type rats (I). Five-µm-thick undecalcified methylmethacrylate sections of ethanol-fixed tibias stained for hPLAP activity and counterstained with nuclear fast red. Bar = 50 µm Fig. 4 shows expression of hPLAP in tissues of wild-type rats transplanted with bone marrow from R26-hPLAP-transgenic rats, 4 weeks post-transplantation. Flow cytometric analysis reveals hPLAP expression (monoclonal antibody clone 8B6) on peripheral blood leukocytes (A) and bone marrow cells (B) in R26-hPLAP bone marrow-transplanted (BMT) wild-type rats (grey area) in contrast to wild-type rats (white area). The staining pattern is very similar to the one observed in R26-hPLAP-transgenic rats (Fig. 3A-B). Lymphocytes in lymph follicles (arrows) and megakaryocytes (arrowheads) in the spleen demonstrate clear hPLAP staining. Bone marrow cells, especially megakaryocytes (arrows), show ubiquitous hPLAP expression, whereas osteoblasts, osteocytes (D, small and large arrowheads, respectively) and chondrocytes in articular cartilage (E) do not express hPLAP, indicating their host origin. In cross-sections of the ileum, enterocytes and smooth muscle cells remain unstained, while cells in the submucosa and endothelial cells show intense hPLAP labelling (F). Five-µm-thick paraffin sections of spleen (C) and ileum (F), and undecalcified methylmethacrylate sections of ethanol-fixed tibias (D-E) stained for hPLAP activity and counterstained with nuclear fast red. Bar = 50 µm.

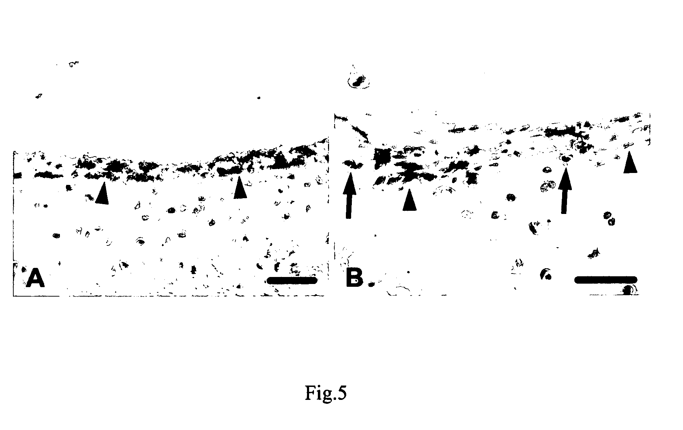

Fig. 5 shows evidence for hPLAP-expressing cells on the articular cartilage after injection of R26-hPLAP-transgenic bone marrow cells into the knee joint of marker tolerant wild-type rats transplanted with R26-hPLAP transgenic bone marrow. One week post-injection, there is a 2 - 3 cells wide layer of donor cells (arrowheads) on the cartilage surface (A). Four weeks post-injection, transgenic donor cells have adopted a fibroblast-like (arrowheads) or chondrocyte-like (arrows) appearance, and show incorporation into the surrounding chondrocyte matrix of host origin (B). Five-µm-thick undecalcified MMA sections of ethanol-fixed knee joints stained for hPLAP activity and counterstained with nuclear fast red. Bar = 50 µm

### EXAMPLES:

A central aspect of this invention is the induction of immunological tolerance to the marker gene. There is a number of known strategies to induce tolerance to foreign antigens. The most robust approach is to lethally irradiate the host organism, and to transplant bone marrow cells expressing non-self antigens (for example the marker gene) into the lethally irradiated host.

Therefore, this approach was used as proof-of-principle in the current invention. Because all immune cells are of donor origin after irradiation and bone marrow transplantation, the foreign donor antigens are not recognised as non-self by immune cells. However, if donor and recipient are not genetically identical (with the exception of the marker gene) this approach requires lifelong immunosuppression because of the danger of a graft-versus-host reaction. The latter situation is, for example, found in leukemia patients after lethal irradiation or chemotherapy and heterologous (between two related, but not genetically identical individuals) bone marrow transplantation. Therefore, we use this approach in the current invention only in genetically identical, inbred or cloned animals. Alternatively, animals can be sublethally irradiated or treated with chemotherapy before bone marrow transplantation, resulting in the generation of chimera. Another strategy is to expose neonatal animals to the foreign antigens. For example, injection of neonates with semi-allogeneic donor cells results in specific tolerance and lifelong survival of skin grafts from the same donor (14). A third strategy is the intravenous infusion of very high numbers of semi-allogeneic donor cells into postnatal hosts (15). In the latter situation, the massive exposure to non-self antigens obviously overrides the immune system so that tolerance is induced. Other methods to induce tolerance to certain antigens are conceivable and could also be used for this invention, for example intranasal application of foreign proteins.

Specifically, the present invention is preferably demonstrated by the application of the following scenarios A to G:
A) Genetic marker in inbred animals (mice, rats, pigs, rabbits, hamster)
   1. Generation of transgenic animals on an inbred genetic background (for example R26-hPLAP-tg Fischer 344 rats in the present experiments, Figs. 1 and 3) expressing the marker gene under a ubiquitous promoter such as beta-actin, cytomegaly virus, or R26, or a promoter targeting transgene (=marker) expression to specific hematopoietic cells such as the megakaryocytic/platelet-specific alphaIIb promoter (16), the lymphoid cell-specific hCD2 promoter (17) or the macrophage-specific c-fms gene promoter (18) .
   2. Generation of marker tolerant wild-type animals of the same inbred strain by a) lethally or sublethally irradiating wild-type animals or treating wild-animals with chemotherapy and b) transplanting the irradiated animals with bone marrow cells from transgenic donors. All hematopoietic but not the mesenchymal and epithelial cells in the recipient are now of transgenic donor origin (Fig. 4).
   3. Transplanting genetically labelled cells from transgenic donors described in 1) into the marker tolerant animals.
   4. Studying the applicability of such cells in cell and gene therapy (Fig. 5).
B) Genetic marker in cloned animals
   This protocol is identical to A) with the difference that genetically identical, cloned animals are used instead of inbred animals.
C) Genetic marker in inbred animals (mice, rats, pigs, rabbits, hamster) using an inducible promoter
   1. Generation of transgenic animals on an inbred genetic background (for example hPLAP-tg Fischer 344 rats in the present experiments) expressing the marker gene under a ubiquitous promoter such as beta-actin, cytomegaly virus, or R26, or a promoter targeting transgene (=marker) expression to specific cell lineages.
   2. Generation of transgenic animals of the same inbred strain expressing the marker transgene under an inducible promoter such as ligand-inducible gene regulatory systems (19). Induction of the transgene, preferably in neonatal animals, results in lifelong tolerance to the marker gene.
   3. Transplanting genetically labelled cells from transgenic donors described in 1) into the marker tolerant animals.
   4. Studying the applicability of such cells in cell and gene therapy.
D) Genetic marker in genetically non-identical animals
   1. Generation of transgenic animals on an outbred or inbred genetic background expressing the marker gene under a ubiquitous promoter such as beta-actin, cytomegaly virus, or R26, or a promoter targeting transgene (=marker) expression to specific cell lineages.
   2. Generation of wild-type animals of the same species but not of the same inbred strain compared with animals described in 1) that are made tolerant to the marker and the antigens of the donor cells by neonatal or postnatal exposition to transgenic cells. Preferably, the animals described in 1) and 2) should be closely related, and should have some common MHC class antigens.
   3. Transplanting genetically labelled cells from transgenic donors described in 1) into the marker tolerant animals described in 2).
   4. Studying the applicability of such cells in cell and gene therapy.
E) Use of autologous cells transduced with the marker gene for cell therapy in marker tolerant animals of any species
   1. Generation of marker tolerant animals by a) taking cells from a neonatal or postnatal animal, b) transducing the cells with the marker gene, and c) re-injecting the transduced cells into the neonatal (preferred) or postnatal (very high cell numbers) host to induce tolerance against the.marker gene.
   2. Transplanting autologous cells from the same animal described in 1) by a) extracting cells from the host, b) transducing these cells with the same marker gene described in 1), and c) transplanting these autologous, transduced cells into the tolerant host.
   3. Studying the applicability of such cells in cell and gene therapy.
F) Use of cells transduced with the marker gene for cell therapy in marker tolerant animals of the same inbred strain
   1. Generation of inbred, marker tolerant animals by a) taking cells from an animal of the same inbred strain, b) transducing the cells with the marker gene, and c) re-injecting the transduced cells into the neonatal (preferred) or postnatal hosts. Alternatively, marker tolerant inbred animals can be obtained by the methods described in A) or C).
   2. Transplanting cells from wild-type animals of the same inbred strain described in 1) by a) extracting cells from these animals, b) transducing these cells with the same marker gene described in 1), and c) transplanting these transduced cells into the tolerant hosts of the same inbred strain.
   3. Studying the applicability of such cells in cell and gene therapy.
      This procedure equals E) with the difference that not autologous cells, but cells of the same inbred strain, are used.
G) Use of cells transduced with the marker gene for cell therapy in marker tolerant cloned animals
   1. Generation of cloned, marker tolerant animals by a) taking cells from an animal of the same clone, b) transducing the cells with the marker gene, and c) re-injecting the transduced cells into neonatal (preferred) or postnatal hosts of the same clone. Alternatively, marker tolerant cloned animals can be obtained by the methods described in B).
   2. Transplanting cells from animals of the same clone described in 1) by a) extracting cells from these animals, b) transducing these cells with the same marker gene described in 1), and c) transplanting these transduced cells into the tolerant hosts of the same clone.
   3. Studying the applicability of such cells in cell and gene therapy.
      This procedure is identical to F) with the difference that not animals from an inbred strain, but cloned animals are used.

It is clear that the studying step is usually connected with killing said model animal or at least disrupting the physiology when assessing the therapy so that a therapeutic effect to the test animal is not reached in the end.

### Example 1.: Injection of bone marrow cells from R26-hPLAP-transgenic inbred Fischer 344 rats into the knee joint of marker tolerant inbred Fischer 344 wild-type rats:

Bone marrow cells from R26-hPLAP-transgenic inbred Fischer 344 rats were injected into the knee joint of marker tolerant inbred Fischer 344 wild-type rats. Tolerance to hPLAP was induced in the wild-type rats by lethal irradiation and bone marrow transplantation with R26-hPLAP-transgenic bone marrow (methodology described in A). One week post-injection, the transgenic BMC had formed a dense, 2 - 3 cells wide, layer on the intact articular cartilage surface (Fig. 5). This finding shows that these cells underwent proliferation on the cartilage surface earlier. After 4 weeks, the genetically labelled cells displayed a more differentiated fibroblast- or chondrocyte-like phenotype and appeared to have become integrated into the surrounding cartilage tissue of wild-type origin. This experiment underscores the potential usefulness and strengths of this model, and clearly demonstrates that mesenchymal precursors obviously present in native bone marrow can adhere to the articular cartilage surface and can, subsequently, differentiate into a fibroblastic or chondrocytic phenotype. Thus, this invention can be used to validate and optimise cell therapies aimed to cure or ameliorate, for example, osteoarthritis.

### Example 2: Myocardial infarction model:

After generation of hPLAP tolerant animals according to protocols A - G, a coronary artery is ligated as a myocardial infarction model. At various time points after the ligation, hPLAP-labelled, cultured mesenchymal stromal cells or more differentiated cells prepared by protocols A - G are intravenously infused. At various time points after the infusion, the animals are killed, and the presence of hPLAP-labelled cells is analysed by histochemistry or immunohistochemistry in the heart. Similar experiments have been described in normal rats with marrow stromal cells expanded in culture and labelled with a reporter gene (20). However, as mentioned above, these experiments are marred by uncertainties regarding immune-mediated rejection phenomena.

### Example 3: Traumatic brain injury model

After generation of hPLAP tolerant animals according to protocols A - G, a traumatic brain injury is induced. At various time points after the injury, hPLAP-labelled, cultured mesenchymal stromal cells or more differentiated cells prepared by protocols A - G are intravenously infused. At various time points after the infusion, the animals are killed, and the presence of hPLAP-labelled cells is analysed by histochemistry or immunohistochemistry in the brain. Similar experiments have been described in normal rats using human bone marrow stromal cells (21). However, as mentioned above, these experiments are marred by uncertainties regarding immune-mediated rejection phenomena.

### REFERENCES

1. Prockop et al., PNAS U S A 2003;100 Suppl 1:11917-23.
2. Gossler et al., In: Joyner (ed.) Gene Targeting: A Practical Approach. New York: NY Oxford Univ. Press, 1993:181-227.
3. Cubitt et al., Trends Biochem Sci 1995;20:448-55.
4. Walter et al., Histochem J 2000;32:99-103.
5. Zagzag et al., Brain Pathol 2003;13:34-7.
6. Kisseberth et al., Dev Biol 1999;214:128-38.
7. Sarukhan et al., J Gene Med 2001;3:260-70.
8. Gross et al., Blood 2003;102:4326-8.
9. Andersson et al., Blood 2003;101:4305-12.
10. Heim et al., Mol Ther 2000;1:533-44.
11. Rosenzweig et al., Blood 2001;97:1951-9.
12. Mujtaba et al., Exp Neurol 2002;174:48-57.
13. Spees et al., Mol Ther 2004;9:747-56.
14. Field et al., Immunol Rev 2001;182:99-112.
15. Ridge et al., Science 1996;271:1723-6.
16. Shi et al., J Thromb Haemost 2004;2:1989-97.
17. de Boer et al., Eur J Immunol 2003;33:314-25.
18. Sasmono et al., Blood 2003;101:1155-63.
19. Albanese et al., Semin Cell Dev Biol 2002;13:129-41.
20. Bittira et al., Eur J Cardiothorac Surg 2003;24:393-8.
21. Mahmood et al., Neurosurgery 2003;53:697-702.

## Claims

1. : Use of marker tolerant animals as a cell therapy or cell-based gene therapy model.

2. : Use according to claim 1, **characterised in that** said marker tolerant animals are lethally irradiated inbred or cloned animals transplanted with bone marrow cells expressing said marker, animals having been exposed neonatally to said marker, animals having been exposed postnatally to cells expressing said marker by intravenous infusion, or animals having been exposed to said marker protein by intranasal application.

3. : Use according to claim 1 or 2, **characterised in that** said marker is Escherichia coli lacZ, green fluorescent protein (GFP), human placental alkaline phosphatase (hPLAP) or neomycin phosphotransferase.

4. : Use according to any one of claims 1 to 3, **characterised in that** said cell therapy or cell-based gene therapy model is used in a cell therapy or cell-based gene therapy for stroke, multiple sclerosis, neurodegenerative disorders, arthroses, intervertebral disc degeneration, ligament injuries, myopathies, myocardial infarction, cardiomyopathies, glomerulonephritis, severe liver cell damages, epidermal cell therapy or cancer.

5. : Use according to any one of claims 1 to 4, **characterised in that** said animal is a rodent, especially a rabbit, a hamster, a mouse, a guinea pig or a rat, a primate, especially a chimpanzee, or a pig.

6. : Use according to any one of claims 1 to 5, **characterised in that** said marker is controlled by an inducible promoter.

7. : Use according to any one of claims 1 to 5, **characterised in that** said marker is controlled by an ubiquitous constitutive promoter, especially R 26, β-actin or β-actin with cytomegalovirus enhancer.

8. : Use according to any one of claims 1 to 7, **characterised in that** said cell therapy or cell-based gene therapy is a therapy employing mesenchymal cells, especially osteoblasts, chondrocytes, adipocytes, fibroblasts, myoblasts or combinations thereof.

9. : Use according to any one of claims 1 to 7, **characterised in that** said cell therapy or cell-based gene therapy is a therapy employing non-hematopoietic cells selected from epithelial cells, endothelial cells, liver cells, pancreas cells, neuronal cells, differentiated or partially differentiated mesenchymal cells, especially mesenchymal cells contained in bone marrow or mesenchymal cells derived from bone marrow stem cells or combinations thereof.

10. : Use according to any one of claims 1 to 7, **characterised in that** said cells with the marker are transplanted into deficient parts of the mammal selected from the group consisting of heart, central nervous system (CNS), bone, cartilage, joint, lung, intestine, liver or pancreas.

11. : Kit for investigating cell therapy or cell-based gene therapy comprising
- a marker tolerant animal and
- a cell comprising the marker.

12. : Kit according to claim 11, **characterised in that** said marker tolerant animal or said cell is defined as in any one of claims 1 to 10.

13. : A method for studying the applicability of cells in cell-therapy and cell-based gene therapy comprising the following steps:
- providing said cells to be studied with a marker,
- transplanting said cells with the marker to an animal, said animal comprising a tolerance against said marker, and
- studying the applicability of said cells with said marker in cell therapy and call-based gene therapy of said marker tolerant animals, preferably comprising killing said animals.

14. : Method according to claim 12 and 13, **characterised in that** said method comprises the steps of
- obtaining a transgenic mammal expressing a marker protein under the control of a promoter,
- isolating bone marrow cells (BMCs) from the transgenic mammal,
- irradiating a wild type mammal of the same species and
- injecting the isolated BMCs into the irradiated wild type mammal,
before transplanting said cells with the marker to the wild type mammal.

15. : Method according to claim 13 or 14, **characterised in that** said cells or said animals is defined in any one of claims 1 to 10.

16. : Animals obtainable by a method according to any one of claims 13 to 15.
